# EUROPEAN PATENT SPECIFICATION

(11) **EP 3 013 272 B1**
(45) Date of publication and mention of the grant of the patent: **18.05.2022**
(21) Application number: 14818220.7
(22) Date of filing: 25.06.2014
(51) Int. Cl.: A61B 18/12, A61B 18/14

(54) **APPARATUS FOR PERFORMING ELECTROSURGERY THROUGH THE USE OF RADIATING ENERGY**
VORRICHTUNG ZUR DURCHFÜHRUNG ELEKTROCHIRURGISCHER EINGRIFFE DURCH VERWENDUNG VON STRAHLUNGSENERGIE
APPAREIL POUR EFFECTUER UNE INTERVENTION ÉLECTROCHIRURGICALE EN UTILISANT UNE ÉNERGIE RAYONNANTE

(30) Priority: 25.06.2013 US 201361839267 P
(43) Date of publication of application: 04.05.2016
(73) Proprietor: Spears, Michael, Houston, Texas 77044 (US)
(72) Inventor: Spears, Michael, Houston, Texas 77044 (US)
(74) Representative: Murgitroyd & Company
(86) International application number: PCT/US2014/044066
(87) International publication number: WO 2014/210136

(56) References cited:
- US-A- 4 674 499
- US-A- 4 674 499
- US-A- 5 895 412
- US-A1- 2005 234 442
- US-A1- 2005 234 442
- US-B1- 6 261 285
- US-B1- 6 582 423
- US-B1- 6 582 423

## Description

### FIELD OF THE DISCLOSURE

The present invention relates to an apparatus for providing a power and grounding pack for use with cutting instruments, herein referred to as "SmartPack," as it works with multiple electrosurgical devices, and also known as "FastPack," indicating that it works under rapid, fast moving, emergency and time critical environments and circumstances. The present invention further relates to cutting and cauterizing biological tissue and or bone. In particular, this power and grounding pack invention relates to a power and grounding source that can be used with electro-surgical cutting device that cuts, cauterizes and ablates, so that a separate grounding pad is not necessary.

### BACKGROUND OF THE INVENTION

Many electrosurgical devices (ESDs) have been developed over the years. One type of ESD includes a handheld cutting or cauterizing element having radio frequency (RF) current applied to it. RF current experiences transmission line effects and losses, and hence, the RF must be carefully impedance matched and field focused in order to get an efficient, narrow field Many of these devices accomplish cutting, coagulation and or cauterizing through use of the conducted heat created at the tip. As the ESD passes high frequency electric current through the cutting tip heat is created which contacts the biological tissue and achieves specific surgical effects, such as cutting, coagulation, or desiccation.

US. Patent No. 4,032738 to Esty et al. teaches a handheld ESD having an on/off switch on the handle, but no impedance matching or field focusing are provided within the hand-held device. Therefore, the efficiency and power of the device are low.

U.S. Patent No. 5,810,809 to Rydell teaches an arthroscopic shaver incorporating cauterization. The shaver portion uses a rotary motor and operates like a Dremel tool (suction removes tissue). The cauterization is accomplished by applying a monopolar RF current to the tubular metal blade or a separate wire. Again, wires are run from an RF source to the cauterizing element, and no impedance matching or field focusing occurs in the handheld device.

US. Patent No. 5,807.392 to Eggers teaches a resistively heated cutting and coagulating tool Some impedance matching is done in the handle of this device, via a transformer and a capacitor. No field focusing is required, since the device generates heat at the tip rather than a focused electromagnetic field

U.S. Patent No. 6,059,781 to Yamanashi, et al. teaches an ESD which cuts and cauterizes via a tip at which RF energy is focused This device includes elements for impedance matching and field focusing. An impedance matching block 52 matches the impedance of the probe 51 with RF generator 44. An impedance matching device is connected to the RF generator and to a Watts/Ampere meter. The meter is connected to a loading and tuning coil. The coil is connected to the surgical instrument via a heavily insulated cable, which is stated to be 110cm long or a multiple of 22. Impedance matching block 52 provides the majority of the impedance matching between the RF generator and the surgical instrument. The patent states in two places that it is desirable not to have a coil in the operative field of the device, as this causes inconvenience to the surgeon. Yamanashi, at al. were not able to design impedance matching and/or field focusing circuitry that would fit within the handheld unit of the invention; hence, they moved it entirely away from the handheld unit area. Unfortunately, both impedance matching and field focusing are dependent upon location and geometry. The field both attenuates and spreads over the distance from the circuitry to the cutting tip, reducing the effectiveness of the surgical device. Therefore, if all of the impedance matching and field focusing could be accomplished by circuitry housed in a hand held unit, this would be a major advantage for surgeons and patients. US2005234442 discloses another electrosurgical cutting device.

Further, improper use of ESDs may expose both the patient and the surgical staff to a number of hazards. There are multiple ESDs out on the market today for use in cutting and cauterizing biological tissue; however, these devices require that a grounding pad be in contact with the subject to complete the electrical circuit, and in order to avoid undesired burns and potentially death of the subject. For instance, a grounding pad must be placed on the patient so that the current may pass to a predictable spot. As a result of misuse or malfunction of such grounding pads, many patients have experienced undesirable burns when the ESD was used to operate on them. Along with undesired burns, electric shock, neuromuscular stimulation, interference with pacemakers and other devices, electrochemical effects from direct currents, implant heating and gas explosions are also hazards involved with the use of ESDs with grounding pads.

Although ESDs with bipolar output terminals (tines at end where cutting is done) do not require grounding pads because the electrical current does not flow through the bulk of the body as it passes between two poles (tines), high power usage of ESDs with monopolar output terminal (probe) requires good electrical contact between a large area of the subject being cut, such as the body (typically the entire back of the patient's body) and the return electrode. If contact with the return path is insufficient, severe burns can occur with areas of poor contact with the return (grounding) pad, or with metal objects in contact with the Earth-ground serving as an unintended (capacitative) return path (such as a hip replacement containing metal). SmartPack provides a purely self-contained return path for the electrons; hence a grounding pad on the patient is not necessary.

There are conventional ESD and generators on the market, having bipolar output terminals and monopolar output terminals; for example, some marketed by Commed/Aspen of Utica, NY; Berchtold of Tuttlingen, Germany; Birtcher of Irvine, Ca; Erbe of Tubingen, Germany; martin of Tuttlingen, Germany and Valleylab of Boulder, CO. An ESD, better known as Bovie is available on the market, but this device touches the tissue, as well as needs a grounding pad Further, the Army has a device that uses a harmonic scalpel; however, it too, needs a grounding pad

Until now, no one has accomplished a cutting field which uses radiated energy produced as electrical current passing through the tip creating an electromagnetic energy field that excites the biological tissue such that a precise cut is produced without the need for the tip to actually contact the biological tissue, thus eliminating the need for a grounding pad This is accomplished through the use of good impedance matching and field focusing with circuitry housed within the control unit and the handheld unit of an ESD. A need, therefore, remains in the art for a system, methods and apparatus for a handheld ESD for cutting and cauterizing, which provides both improved impedance matching and field focusing, and that does not require a grounding pad

### SUMMARY OF THE INVENTION

In accordance with the present invention, the above and other problems are solved by an apparatus providing power and grounding to an electrosurgical device (ESD) for use in operations. The invention is defined in appended independent claim 1, preferred embodiments are described in the dependent claims.

Further, the ESD of the present invention behaves exactly as a radiating antenna operating at 13.56MHz in the near field, and it concentrates the RF energy coming from the RF Module into the probe tip. The electric field of the radiating electro-magnetic energy becomes very intense on pointing tips and sharp edges. When a high intensity electric field comes in close proximity to a biological tissue, it induces ionic vibration of the free ions naturally present on such tissue. The ionic vibration generates intracellular heat, which causes the cells to boil and eventually explode, thus resulting in a cutting effect of the tissue. If the electric field energy is moderated with PWM techniques, it causes dehydration and/or fulguration of the cells resulting in coagulation of the biological tissue. The functioning principle of the ESD is similar to a microwave oven that excites the molecules of water present on most foods. The electro-magnetic energy emanating from inside the microwave oven generates heat, which causes the food temperature to rise. Thus, unlike the ESD commonly known as the Bovie and other ESDs on the market, there is no need for the ESD tip to physically touch the tissue of the subject in order for cutting to occur since the electrical charge does not actually pass-through the biological tissue..

Other advantages exist with SmartPack, such as un-tethered power generation, which is desirable to power ESDs, especially out in the field, emergency situations or in war-torn/combat environments, where standard grid-based power is not readily available, SmartPack may include a method of internal power generation at the SmartPack. Such generation includes, but is not limited to, the use of 14.4 volt lithium ion batteries for power generation by the SmartPack. There is also a power regulation method to ensure the proper amount of power to the electro-surgical device and that the power, generated by the batteries, is consistent throughout usage of the device.

Another advantage is that a compressed inert gas can be transferred through the system to the tip of the specially created ESD for achieving submergibility of the tip in fluids, including a saline-based environment, and further for clearing debris, such as fluids or tissue from the cutting area, thus insulating the tip during operation.

These and various other features, as well as advantages, which characterize the present invention, will be apparent from a reading of the following detailed description and a review of the associated drawings.

### BRIEF DESCRIPTION OF THE DRAWINGS AND PICTURES

FIG 1A. is a block diagram depicting the simplified sections of the RF scalpel circuitry.
FIG 1B. is a block diagram depicting the detailed description of the RF Scalpel circuitry.
FIG. 2. shows a DC/DC Converter simplified SEPIC topology.
FIG. 3. shows an RF Module simplified Class E inverter topology.
FIG. 4. Simplified schematic of the RF Probe.
FIG. 5. DC/DC Converter complete schematic.
FIG. 6. RF Module Schematic

### DETAILED DESCRIPTION OF EMBODIMENTS

According to one embodiment of the invention, an apparatus is provided for electro-surgery, wherein the apparatus does not have to contact the tissue of the subject for cutting to occur and a grounding pad is not required for usage of the ESD as no energy is passed from the device through the subject.

In one embodiment of the invention, electro-magnetic energy from an RF power source may be used to produce a cut and/or cauterization of living organic tissue. The energy is delivered via a special probe that radiates electro-magnetic energy in close proximity to the living organic tissue without direct contact with the tissue. Since the tip does not actually come in contact with tissue, the capacitance across the gap is part of the circuit and instead of the excess energy being passed through to the body of the subject, the energy is returned back through the hand piece of the ESD, which is connected to the SmartPack.

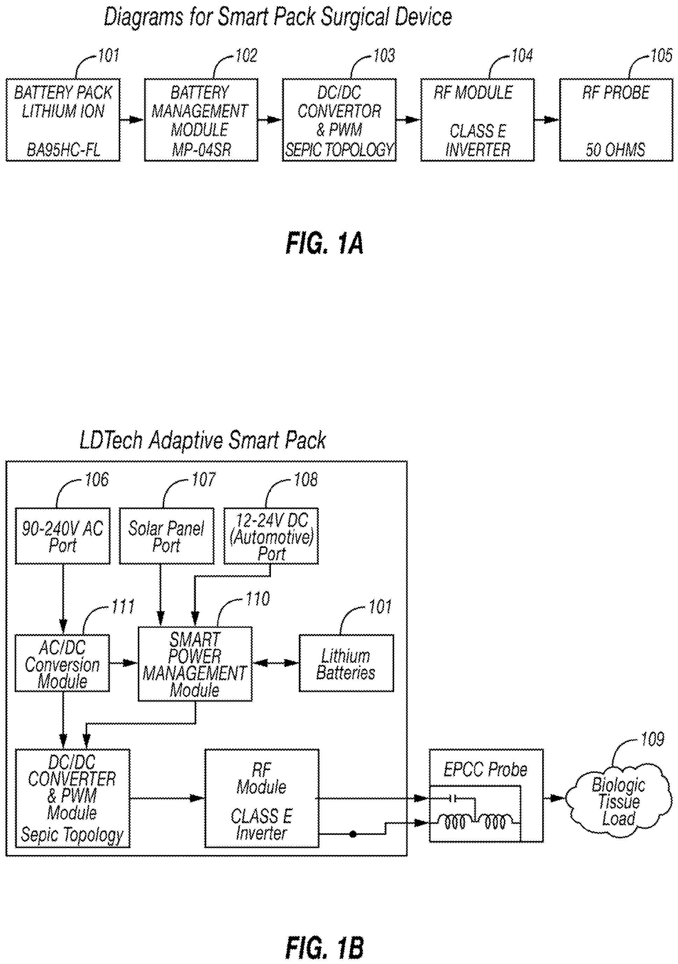

Turning now to FIG. 1A and 1B, shown are block diagrams of the functional components of an embodiment of the SmartPack.

FIG. 1A shows an embodiment were standard Lithium Batteries 101 are used. The SmartPack can be fully powered by standard Lithium batteries 101. The battery pack utilizes up to four Lithium-Ion individual batteries to generate the RF energy. Each battery has imbedded circuitry that monitors the charge/discharge cycles, as well as providing information regarding battery status. The Lithium batteries are rechargeable from external sources, including wall outlet, solar panels, or automotive battery

FIG. 1B also shows a 90-240 VAC Port 106. This is a universal wall power input jack that will allow the user to charge the batteries, such as the lithium batteries, or directly operate the system from grid power. Not shown is the ability to use nano-based power sources, such as micro-batteries and ultra-capacitors as a means of a power source in the SmartPack, or in the alternative a self-contained hand piece. In such an instance, all of the circuitry shown in the FIGs may be self-contained in the hand piece eliminating the need for a separate power unit altogether, e.g. an ESD that does not require a cable or an external power unit.

The Solar Panel Port 107 is provided for the option to charge the batteries from an optional solar panel that will plug into the unit. The 12-24 VDC Port 108 as a port for charging the batteries or operating SmartPack from an optional automotive power jack. The ability to handle 12 to 24 VDC 108 allows utilization of most vehicles for battery charging and operation, including Humvees and other military vehicles.

The Smart Power Management Module 110 "also known as Battery Management Module 102 of FIG. 1A" is a fully autonomous power management system that controls all aspects of powering the system. It handles the power switching between battery power and power from an external supply along with all aspects regarding charging and discharging of the batteries. This allows for powering the system while charging the batteries at the same time.

The AC/DC Conversion Module 111 of FIG.18 is shown, and will accept universal AC voltage ranging from 90 VAC to 240 VAC and 50Hz to 60Hz and convert the input AC voltage into the system input DC voltage level The DC voltage can be used to either power the unit directly or to recharge the batteries.

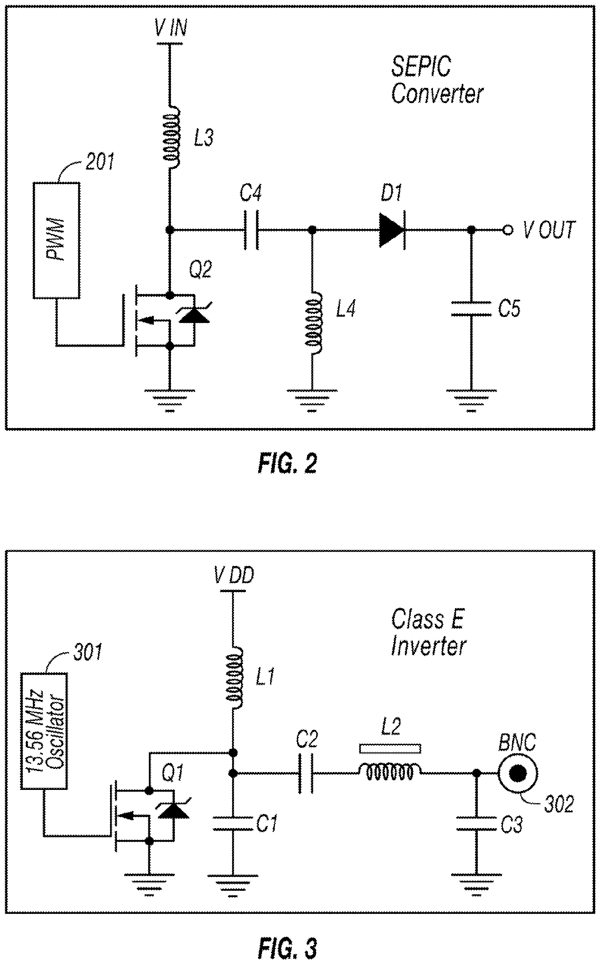

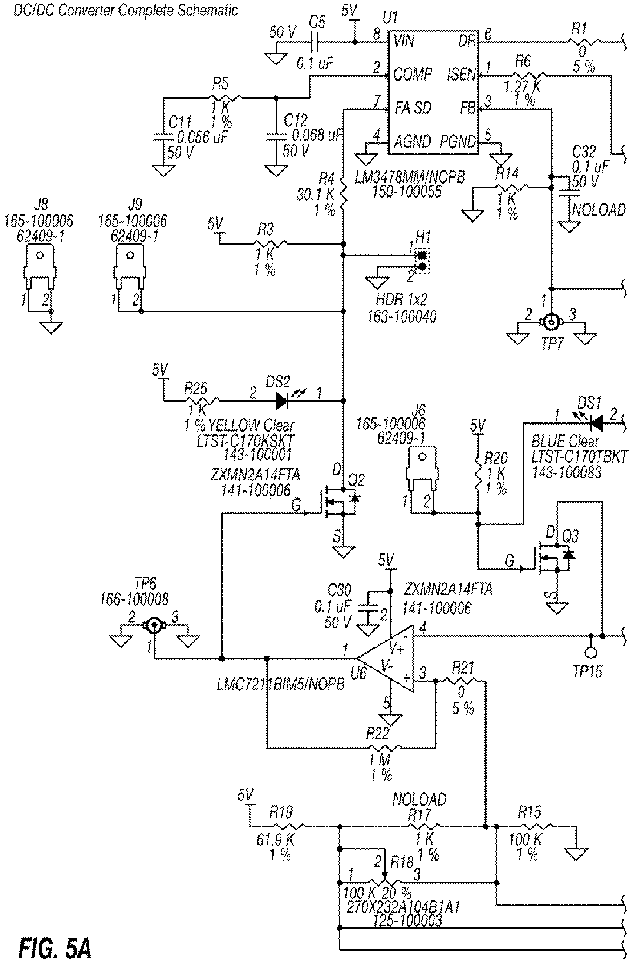

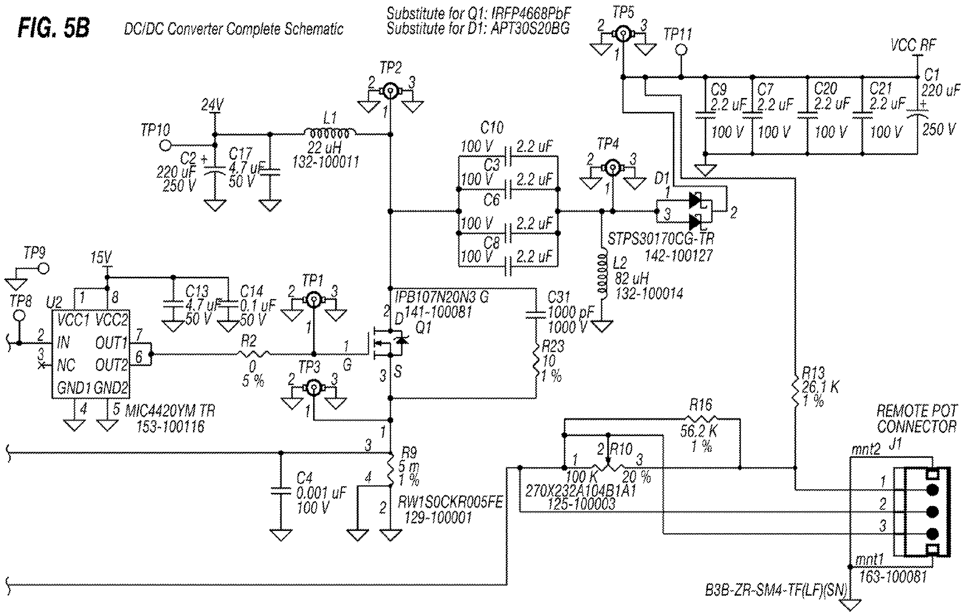

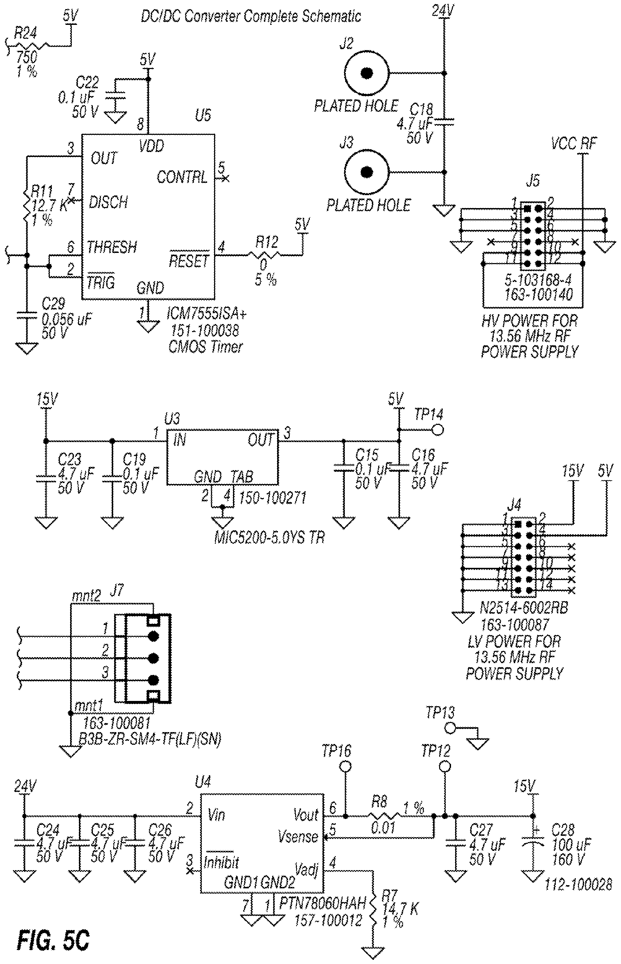

Looking now to FIG. 2 the DC/DC Converter & Pulse-Width Modulation (PWM) Module. The DC/DC converter is a custom design switching regulator utilizing the traditional single ended primary inductor converter (SEPIC) topology to generate appropriate system voltages. The function of the DC/DC Converter is to regulate the amount of power delivered to the RF Probe through the RF Module, It accomplishes that by taking power from the battery or external power source through the Battery Management Module 102, and varying its DC output voltage from 34V to 100V. It also uses PWM 201 techniques to modulate the output voltage from 0 V to a pre-determined fixed voltage and frequency, and duty cycle from 10% to 90% range. When the converter outputs a DC constant voltage, the system is in cutting mode. When the converter outputs a PWM voltage, the system is in cauterization mode. This is further shown in FIG. 5.

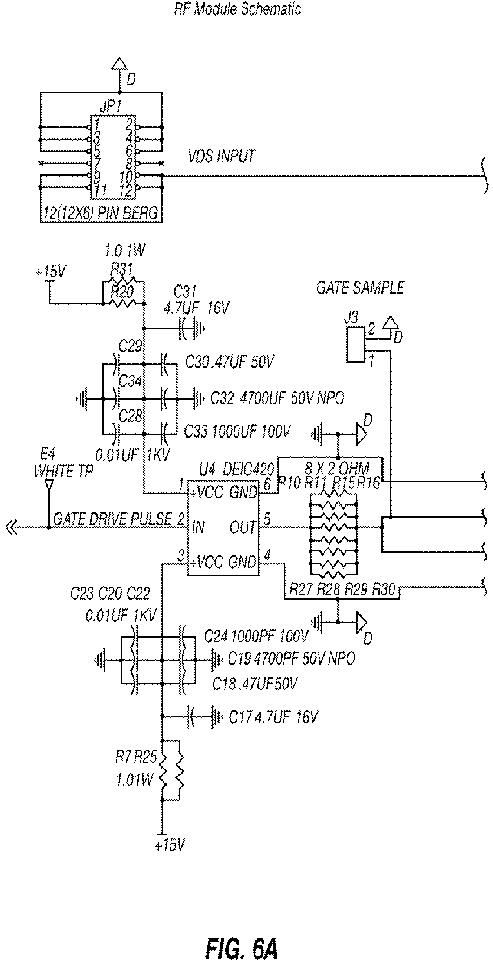

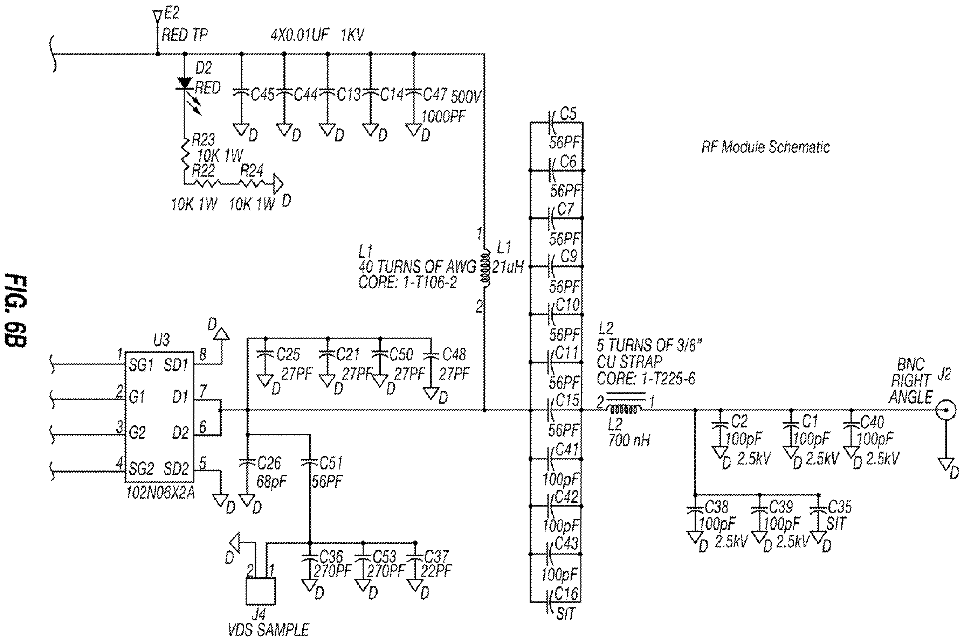

FIG. 3 displays the RF Module of the SmartPack which converts the DC output voltage provided by the DC/DC Converter (constant or PWM) into a sine wave oscillating at the precise frequency of 13.56 MHz 301 for this embodiment. This sine wave output is fed into a BNC connector 302 which is connected to a 50 ohms coax cable connecting the RF probe. The power of the sine wave is directly proportional to the DC input voltage to the RF Module coming from the DC/DC Converter FIG. 2. As shown in FIG. 3, the RF Module utilizes a typical Class E inverter that converts DC power which is used to power the RF Module 104, which is easily transmitted through a cable or any form of transmission line. To produce a reliable cutting and/or cauterization action on a living tissue, the RF Module produces in excess of 130W of power driving 50 ohms impedance. The configuration of the RF Module is further shown in FIG. 6.

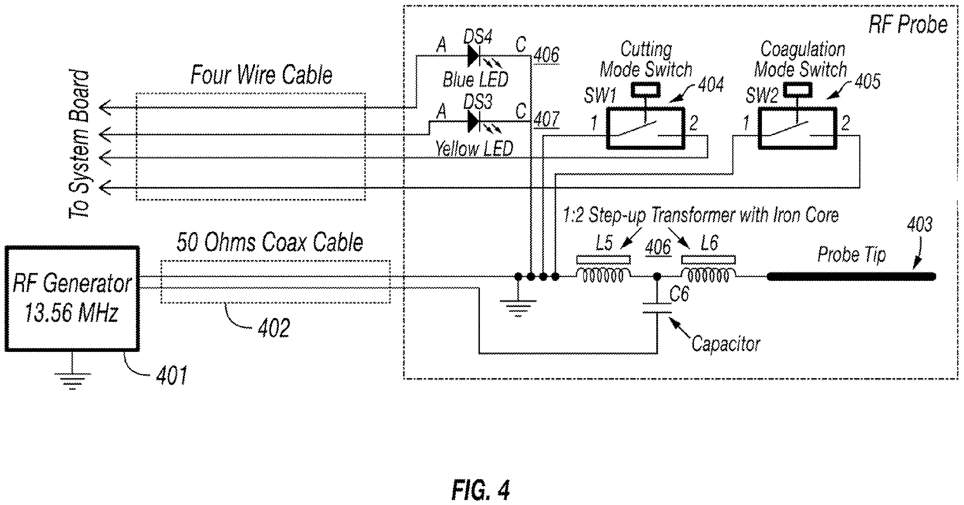

Now turning to FIG. 4 which shows the specially created monopolar RF Probe to be used with SmartPack. The RF Probe is comprised of an impedance matching network and an iron core 406, and a tip 403 as depicted on FIG. 4. It is connected to the RF generator 401 through a 50 ohms coax cable 402, which is impedance matched to the probe. In addition, the probe contains two push-button switches 404, 405 to select cutting or cauterizing mode, and two individual LEDs 406, 407 to indicate the operating mode status.

The special ESD for the SmartPack is an electromagnetic precision convergence cutter. Power is generated by a fixed 13.56MHz, 50 ohm battery operated generator or SmartPack. Power is transferred down the center conductor wire of shielded coaxial cable with a characteristic impedance of 50 ohms. A second and third shielded center conductor is sleeved with the main power center conductor. The second and third center conductor controls the SPST physical on/off switch to activate or deactivate power for cutting mode and power for coagulation mode being transferred to the tissue (load).

Notably in FIG. 4, the present invention differs from the prior art in that it uses a carbonyl iron powder material core 406 within the coil of the hand piece. At 13.56 MHz, Carbonyl E has been noticed as the most efficient with the highest permeability for focusing pinpoint energy at the tip of the hand piece. The carbonyl iron powder material increases permeability and magnetic flux density by concentrating the magnetic flux lines of force. Further, potentiometers are used for optimizing power transfer, as the impedance changes due to physical changes in the biological tissue or the environment and keeps the power constant.

Another embodiment contains a port and means to connect compressed air to the SmartPack and or RF Probe. If the compressed air is connected through the SmartPack then there will be an additional connection and hose going from the SmartPack to the RF Probe to allow the compressed air to be released at the tip of the probe. By using compressed air the RF Probe may be used in environments were other ESD's probe cannot. Compressed air will remove fluids and debris from the path of the cutting probe allowing for the appropriate energy transfer.

A final embodiment contains additional charging/powering ports into the Power Management Module for powering and charging the system. These ports can include but are not limited to USB port, and positive and negative terminal leads for electrical connections, such as use with jumper cables.

Based on the foregoing, it should be appreciated that the various embodiments of the invention include many different working parts, which make up the apparatus for providing power and grounding for electro-surgery. It will be apparent by those skilled in the art that various modifications or variations may be made in the present invention without departing from the scope of the invention. Other embodiments of the present invention will be apparent to those skilled in the art from consideration of the specification and practice of the invention disclosed herein.

## Claims

1. An apparatus for electro-surgery, comprising:
a radio frequency generator (104);
a DC/DC converter (103);
a pulse-wave modulator (103) ;
at least one battery (101);
a power supply manager (110);
an AC/DC converter (111);
an AC port (106);
a solar panel port (107);
an automotive port (108); and
a probe port, wherein the power supply manager receives power from the solar panel port or the automotive port or the at least one battery and ensures a threshold level of power is provided to the DC/DC converter such that the output of the DC/DC converter is 34-100V, the power from the power supply manager is provided to radio frequency generator through the DC/DC converter or the pulse-wave modulator and the radio frequency generator generates an electromagnetic field in excess of 130W at 50 ohms and 13.56 MHz, which is provided to the probe port, optionally wherein the threshold level of power is designated by a user.

2. The apparatus as claimed in claim 1, wherein (i) when the power from the power supply manager is provided through DC/DC converter, a probe connected to the probe port is in cut mode, or (ii) when the power from the power supply manager is provided through pulse-wave modulator, a probe connected to the probe port is in cauterize mode.

3. The apparatus of claim 1, wherein the power supply manager receives power from the AC port through the AC/DC converter and charges the at least one battery.

4. The apparatus as claimed in claim 3, wherein (i) the power from the power supply manager is provided to the radio frequency generator or the pulse-wave modulator and the radio frequency generator generates an electromagnetic field, which is provided to the probe port or (ii) said power from the AC port is provided to the radio frequency generator through the DC/DC converter or the pulse-wave modulator and the radio frequency generator generates an electromagnetic field, which is provided to the probe port.

5. The apparatus as claimed in claim 1, further comprising: a radio frequency probe, which is impedance matched to the radio frequency generator.

6. An apparatus of claim 1, further comprising:
a hand held unit housing;
means for providing electromagnetic energy to the handheld unit housing;
means for providing a switch signal from the housing to a power supply;
a cutting tip emerging from the housing, from which radiating energy is produced by electromagnetic field that is generated;
a switch on the housing for generating the switch signal; and
circuitry for impedance matching between a radio frequency module, the housing, the cutting tip and a biological tissue.

7. The apparatus of claim 6, wherein compressed air can be transferred to the tip wherein the compressed air in use is inserted into the apparatus and connected to the tip such that the compressed air is released at the tip such that in use fluids and debris are removed from the path of the tip and allowing for appropriate energy transfer.

8. The apparatus of either of claims 6 or 7, wherein the cutting tip comprises a carbonyl iron powder material core.

9. The apparatus of claim 8, wherein the carbonyl iron powder material core is a Carbonyl E iron powder material core.

## Patentansprüche

1. Eine Vorrichtung zur Elektrochirurgie, die Folgendes beinhaltet:
einen Hochfrequenzgenerator (104);
einen DC/DC-Wandler (103);
einen Impulswellenmodulator (103);
mindestens eine Batterie (101);
einen Stromversorgungsmanager (110);
einen AC/DC-Wandler (111);
einen AC-Anschluss (106);
einen Solarpaneelanschluss (107);
einen Fahrzeuganschluss (108); und
einen Sondenanschluss, wobei der Stromversorgungsmanager Strom von dem Solarpaneelanschluss oder dem Fahrzeuganschluss oder der mindestens einen Batterie empfängt und sicherstellt, dass dem DC/DC-Wandler ein Schwellenwert an Strom bereitgestellt wird, sodass die Ausgabe des DC/DC-Wandlers 34-100 V beträgt, der Strom von dem Stromversorgungsmanager dem Hochfrequenzgenerator durch den DC/DC-Wandler oder den Impulswellenmodulator bereitgestellt wird und der Hochfrequenzgenerator ein elektromagnetisches Feld über 130 W bei 50 Ohm und 13,56 MHz erzeugt, das dem Sondenanschluss bereitgestellt wird, wobei der Schwellenwert an Strom optional durch einen Benutzer bestimmt wird.

2. Vorrichtung gemäß Anspruch 1, wobei (i) wenn der Strom von dem Stromversorgungsmanager durch den DC/DC-Wandler bereitgestellt wird, sich eine Sonde, die mit dem Sondenanschluss verbunden ist, im Schnittmodus befindet, oder (ii) wenn der Strom von dem Stromversorgungsmanager durch den Impulswellenmodulator bereitgestellt wird, sich eine Sonde, die mit dem Sondenanschluss verbunden ist, im Ätzmodus befindet.

3. Vorrichtung gemäß Anspruch 1, wobei der Stromversorgungsmanager durch den AC/DC-Wandler Strom von dem AC-Anschluss empfängt und die mindestens eine Batterie lädt.

4. Vorrichtung gemäß Anspruch 3, wobei (i) der Strom von dem Stromversorgungsmanager dem Hochfrequenzgenerator oder dem Impulswellenmodulator bereitgestellt wird und der Hochfrequenzgenerator ein elektromagnetisches Feld erzeugt, das dem Sondenanschluss bereitgestellt wird, oder (ii) der Strom von dem AC-Anschluss dem Hochfrequenzgenerator durch den DC/DC-Wandler oder den Impulswellenmodulator bereitgestellt wird und der Hochfrequenzgenerator ein elektromagnetisches Feld erzeugt, das dem Sondenanschluss bereitgestellt wird.

5. Vorrichtung gemäß Anspruch 1, die ferner Folgendes beinhaltet: eine Hochfrequenzsonde, die in der Impedanz an den Hochfrequenzgenerator angepasst ist.

6. Vorrichtung gemäß Anspruch 1, die ferner Folgendes beinhaltet:
ein handgeführtes Einheitsgehäuse;
Mittel zum Bereitstellen von elektromagnetischer Energie an das handgeführte Einheitsgehäuse;
Mittel zum Bereitstellen eines Schaltsignals von dem Gehäuse zu einer Stromversorgung;
eine Schneidplatte, die aus dem Gehäuse austritt, von der Strahlungsenergie durch das elektromagnetische Feld, das erzeugt wird, produziert wird;
einen Schalter auf dem Gehäuse zum Erzeugen des Schaltsignals; und
einen Schaltkreis zur Impedanzanpassung zwischen einem Hochfrequenzmodul,
dem Gehäuse, der Schneidplatte und einem biologischen Gewebe.

7. Vorrichtung gemäß Anspruch 6, wobei Druckluft zu der Platte transferiert werden kann, wobei die Druckluft bei Verwendung in die Vorrichtung eingeführt und mit der Platte verbunden wird, sodass die Druckluft so an der Platte freigegeben wird, dass bei Verwendung Fluide und Ablagerungen aus dem Pfad der Platte entfernt werden und ein geeigneter Energietransfer ermöglicht wird.

8. Vorrichtung gemäß Anspruch 6 oder 7, wobei die Schneidplatte einen Kern aus Pulvermaterial aus Carbonyleisen beinhaltet.

9. Vorrichtung gemäß Anspruch 8, wobei der Kern aus Pulvermaterial aus Carbonyleisen ein Kern aus Pulvermaterial aus Carbonyl-E-Eisen ist.

## Revendications

1. Un appareil pour une électrochirurgie, comprenant :
un générateur de radiofréquence (104) ;
un convertisseur CC/CC (103) ;
un modulateur d'ondes d'impulsions (103) ;
au moins une batterie (101) ;
un gestionnaire d'alimentation en puissance (110) ;
un convertisseur CA/CC (111) ;
un port CA (106) ;
un port pour panneau solaire (107) ;
un port automobile (108) ; et
un port pour sonde, dans lequel le gestionnaire d'alimentation en puissance reçoit de la puissance provenant du port pour panneau solaire ou du port automobile ou de l'au moins une batterie et garantit qu'un niveau seuil de puissance est fourni au convertisseur CC/CC de telle sorte que la sortie du convertisseur CC/CC est de 34 à 100 V, la puissance provenant du gestionnaire d'alimentation en puissance est fournie au générateur de radiofréquence par l'intermédiaire du convertisseur CC/CC ou du modulateur d'ondes d'impulsions et le générateur de radiofréquence génère un champ électromagnétique dépassant 130 W à 50 ohms et 13,56 MHz, lequel est fourni au port pour sonde, facultativement dans lequel le niveau seuil de puissance est désigné par un utilisateur.

2. L'appareil tel que revendiqué dans la revendication 1, dans lequel (i) lorsque la puissance provenant du gestionnaire d'alimentation en puissance est fournie par l'intermédiaire du convertisseur CC/CC, une sonde raccordée au port pour sonde est en mode découpe, ou (ii) lorsque la puissance provenant du gestionnaire d'alimentation en puissance est fournie par l'intermédiaire du modulateur d'ondes d'impulsions, une sonde raccordée au port pour sonde est en mode cautérisation.

3. L'appareil de la revendication 1, dans lequel le gestionnaire d'alimentation en puissance reçoit de la puissance provenant du port CA par l'intermédiaire du convertisseur CA/CC et charge l'au moins une batterie.

4. L'appareil tel que revendiqué dans la revendication 3, dans lequel (i) la puissance provenant du gestionnaire d'alimentation en puissance est fournie au générateur de radiofréquence ou au modulateur d'ondes d'impulsions et le générateur de radiofréquence génère un champ électromagnétique, lequel est fourni au port pour sonde ou (ii) ladite puissance provenant du port CA est fournie au générateur de radiofréquence par l'intermédiaire du convertisseur CC/CC ou du modulateur d'ondes d'impulsions et le générateur de radiofréquence génère un champ électromagnétique, lequel est fourni au port pour sonde.

5. L'appareil tel que revendiqué dans la revendication 1, comprenant en outre : une sonde de radiofréquence, laquelle est adaptée en impédance au générateur de radiofréquence.

6. Un appareil de la revendication 1, comprenant en outre :
un boîtier d'unité à main ;
un moyen pour la fourniture d'énergie électromagnétique au boîtier d'unité à main ;
un moyen pour la fourniture d'un signal d'interrupteur du boîtier à une alimentation en puissance ;
une pointe de découpe émergeant du boîtier, de laquelle de l'énergie rayonnante est produite par un champ électromagnétique qui est généré ;
un interrupteur sur le boîtier pour la génération du signal d'interrupteur ; et
une circuiterie pour une adaptation d'impédance entre un module de radiofréquence, le boîtier, la pointe de découpe et un tissu biologique.

7. L'appareil de la revendication 6, dans lequel de l'air comprimé peut être transféré à la pointe, l'air comprimé, lors de l'utilisation, étant inséré dans l'appareil et raccordé à la pointe de telle sorte que l'air comprimé est libéré au niveau de la pointe de telle sorte que, lors de l'utilisation, des fluides et des débris sont retirés de la trajectoire de la pointe et un transfert d'énergie approprié est permis.

8. L'appareil de l'une ou l'autre des revendications 6 et 7, dans lequel la pointe de découpe comprend un noyau en matériau en poudre de fer carbonyle.

9. L'appareil de la revendication 8, dans lequel le noyau en matériau en poudre de fer carbonyle est un noyau en matériau en poudre de fer carbonyle E.
